Europäisches Patentamt

European Patent Office

Office européen des brevets

(19)

(11) Publication number: **0 341 100 B1**

(12) **EUROPEAN PATENT SPECIFICATION**

(45) Date of publication of patent specification: **16.03.94**    (51) Int. Cl.5: **C12N 15/62**, **A61K 37/24**

(21) Application number: **89400912.5**

(22) Date of filing: **03.04.89**

(54) **DNA coding for thymosin and TNF.**

(30) Priority: **03.04.88 JP 81683/88**

(43) Date of publication of application:
**08.11.89 Bulletin 89/45**

(45) Publication of the grant of the patent:
**16.03.94 Bulletin 94/11**

(84) Designated Contracting States:
**AT BE CH DE ES FR GB GR IT LI LU NL SE**

(56) References cited:
**EP-A- 0 155 549**
**EP-A- 0 247 906**

**BIOCHEMISTRY INTERNATIONAL, vol. 18, no. 3, March 1989, pages 501-508, Academic-Press Australia, AU; Y. TSUJI et al.: "Production in Escherichia coli of humanthymosin beta, as chimeric protein with human tumor necrosis factor"**

(73) Proprietor: **Mizuno, Den'Ichi**
**Okamoto-18**
**Kamakura City, Kanagawa(JP)**

Proprietor: **Soma, Gen-Ichiro**
**1-10-21, Higashi-Tamagawa**
**Setagaya-ku, Tokyo(JP)**

(72) Inventor: **Mizuno, Den'ichi**
**Okamoto-18**
**Kamakura**
**Kanagawa(JP)**
Inventor: **Soma, Gen-Ichiro**
**Higashi-Tamagawa 1-10-21**
**Setagaya Tokyo(JP)**
Inventor: **Tsuji, Yoshiaki Biotechnology Research Center**
**Teikyo University**
**1091**
**Sun Sawa-arashi**
**Sagamiko-cho, Tsukui-gun Kanagawa(JP)**

(74) Representative: **Martin, Jean-Jacques et al**
**Cabinet REGIMBEAU**
**26, Avenue Kléber**
**F-75116 Paris (FR)**

## Description

Reference is made to Ser. No.86110139.2 filed by the same applicants as of the present application, entitled "Anti-Tumor Polypeptides and A Method of Preparing The Same", and to Ser.No.87400261 filed by the same applicants as of the present application, entitled "Novel DNAS and Processes for Their Preparation, Novel Plasmids Possessing Them, Novel Polypeptides and Processes for Their Preparation and Novel Anti-tumor Agents Comprising Said Polypeptides."

Field of the Invention

This invention relates to DNAs. More particularly, it is concerned with DNAs coding both thymosin $\beta_4$ - (hereunder referred to as TH$\beta_4$) and TNF and processes for their preparation, plasmids comprising them, polypeptides which are the expression products of said DNAs and processes for said polypeptides, and anti-tumor agents comprising said polypeptides.

Description of the Prior Art

TH$\beta_4$ is a thymus hormone reported to revive immunity [Modern Chemistry (Gendai Kagaku). Dec., 34-38, 1985]. Thus it is guessed to be capable of inducing differentiation of malignant bone marrow cells such as malignant tumors into monocytes. Its amino acid sequence is found to be as follows:

Ser-Asp-Lys-Pro-Asp-Met-Ala-Glu-Ile-Glu-Lys-Phe-Asp-Lys-

Ser-Lys-Leu-Lys-Lys-Thr-Glu-Thr-Gln-Glu-Lys-Asn-Pro-Leu-

Pro-Ser-Lys-Glu-Thr-Ile-Glu-Gln-Glu-Lys-Gln-Ala-Gly-Glu-Ser

According to the prior art processes, however, the maximum yield is only 4.1 $\mu$g from 1 g of cattle thymus, and the purification procedures are very complicated (Annals. New York Academy of Sciences, 249, p.134, 1975). In conclusion, the utility has not been very much researched because of no processes for the preparation of TH$\beta_4$ of high purity on a large scale.

In turn, TNF is a general term for tumor necrosis factors, and the most popular human-originated one is TNF-$\alpha$ obtained from human cell HL-60 (ATCC 240) described in "The Journal of Biol. Chem.", 260, pp. 2345-2354, 1986. The amino acid sequence of this polypeptide has been elucidated to be as follows:

Val-Arg-Ser-Ser-Ser-Arg-Thr-Pro-Ser-Asp-Lys-Pro-Val-Ala-His-

Val-Val-Ala-Asn-Pro-Gln-Ala-Glu-Gly-Gln-Leu-Gln-Trp-Leu-Asn-

Arg-Arg-Ala-Asn-Ala-Leu-Leu-Ala-Asn-Gly-Val-Glu-Leu-Arg-Asp-

Asn-Gln-Leu-Val-Val-Pro-Ser-Glu-Gly-Leu-Tyr-Leu-Ile-Tyr-Ser-

Gln-Val-Leu-Phe-Lys-Gly-Gln-Gly-Cys-Pro-Ser-Thr-His-Val-Leu-

Leu-Thr-His-Thr-Ile-Ser-Arg-Ile-Ala-Val-Ser-Tyr-Gln-Thr-Lys-

EP 0 341 100 B1

```
Val-Asn-Leu-Leu-Ser-Ala-Ile-Lys-Ser-Pro-Cys-Gln-Arg-Glu-Thr-
Pro-Glu-Gly-Ala-Glu-Ala-Lys-Pro-Trp-Tyr-Glu-Pro-Ile-Tyr-Leu-
Gly-Gly-Val-Phe-Gln-Leu-Glu-Lys-Gly-Asp-Arg-Leu-Ser-Ala-Glu-
Ile-Asn-Arg-Pro-Asp-Tyr-Leu-Asp-Phe-Ala-Glu-Ser-Gly-Gln-Val-
Tyr-Phe-Gly-Ile-Ile-Ala-Leu
```

In the above amino acid sequence, the segment starting with the eighteenth amino acid Ala and ending with the last amino acid Leu has the same sequence which may be constructed by linking guanine (G) with the beginning of the base sequence of the fourth exon of TNF-α. So, hereunder throughout the specification, said segment Ala-Leu will be referred to as "the amino acid sequence corresponding to the amino acid sequence constructed by linking guanine with the beginning of the base sequence of the fourth exon of TNF-α." TNF-α is, however, cytotoxic to only few cells (Science, 230, 943-945, 1985), and further is reported to cause so-called cachexy which promotes dissimilation of fat cells (Therapeutic Research, 7, vol. 2, 184-190, 1987). For this there have been provided some recombinant TNF (hereunder referred to as rTNF) which are expected to be free from the defects of TNF-α.

Brief Summary of the Invention

Accordingly, it is the object of the present invention to provide novel DNAs which encode both THβ4 and TNF and enabled the preparation of the former of high purity on a large scale for the first time and processes for their preparation, novel plasmids comprising said DNAs, novel polypeptides which are the expression products of said DNAs and processes for their preparation, and novel anti-tumor agents comprising said polypeptides.

Brief Description of the Drawings

Fig. 1 shows the base sequence of XhoI/PstI segment of the genome gene of the prior art anti-tumor polypeptide from THP-I cells.
Fig. 2 is a graph showing the elution pattern in the first chromatography on Pharmacia mono Q FPLC column of the supernatant from centrifugation in a working example which contains a polypeptide of the present invention.
Fig. 3 is a graph showing the elution pattern of active fractions collected in view of the elution pattern shown in Fig. 2 when they were applied to the second chromatography on Pharmacia mono Q FPLC column.
Fig. 4 is a graph exhibiting anti-tumor activity to Meth A fibrosarcoma of some polypeptides within or outside the scope of the present invention.
Fig. 5 is a graph exhibiting anti tumor activity to Lewis lung carcinoma (3LL) of some polypeptides within or outside the scope of the present invention.
Fig. 6 is a graph exhibiting anti-tumor activity to MH134 tumor of some polypeptides within or outside the scope of the present invention.
Fig. 7 is a graph exhibiting anti-tumor activity to Bl6 melanoma of some polypeptides within or outside the scope of the present invention.
Fig. 8 is a graph exhibiting effects of some polypeptides within or outside the scope of the present invention on the production of endogenous TNF.

Detailed Description of the Invention

According to the present invention, there are provided novel DNAs encoding the following amino acid sequence:
(The amino acid sequence of THβ4)-Asp-Pro-x-(the amino acid sequence corresponding to the base sequence constructed by linking guanine with the beginning of the base sequence of the fourth exon of TNF-α )
wherein X is a part of the linkage or represents any of the following amino acid sequences:

3

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; and

5) Val-Arg-Ser-Cys-Thr-Arg-Thr-Pro-Ser-Arg-Lys-Pro-Val-Ala-His-Val-Val.

The novel DNAs of the present invention may be prepared by combining DNA encoding the amino acid sequence of TH$\beta_4$ with another DNA encoding the sequence: x-(the amino acid sequence corresponding to the base sequence constructed by linking guanine with the beginning of the base sequence of the fourth exon of TNF-$\alpha$ through an Asp-Pro linkage. The thus-prepared DNAs of the present invention may be expressed by being incorporated into an appropriate vector DNA in an expressible manner followed by transformation of host organisms including animal cells, yeasts, B. subtilis, E. coli and the like with the resulting rDNAs.

Their expression product polypeptides may be decomposed by the contact with an appropriate acid such as formic acid or acetic acid at 37°C for 24-48 hours to provide TH$\beta_4$ and x-(the amino acid sequence corresponding to the base sequence constructed by linking guanine with the beginning of the base sequence of the fourth exon of TNF-$\alpha$ wherein x is defined as in the above.

(1) Preparation of DNAs encoding (the amino acid sequence of TH$\beta_4$)-Asp-Pro-x-(the amino acid sequence corresponding to the base sequence constructed by linking guanine with the beginning of the base sequence of the fourth exon of TNF-$\alpha$)

The DNAs of the present invention may be prepared in any of the following three manners:

1) DNA encoding (the amino acid sequence of TH$\beta_4$)-Asp-Pro is linked with DNA coding x-(the amino acid sequence corresponding to the base sequence constructed by linking guanine with the beginning of the base sequence of the fourth exon of TNF-$\alpha$);

2) DNA encoding (the amino acid sequence of TH$\beta_4$) is linked with Asp-Pro-x-(the amino acid sequence corresponding to the base sequence constructed by linking guanine with the beginning of the base sequence of the fourth exon of TNF-$\alpha$): or

3) DNA encoding (the amino acid sequence of TH$\beta_4$), DNA encoding Asp-Pro and DNA encoding x- (the amino acid sequence corresponding to the base sequence constructed by linking guanine with the beginning of the base sequence of the fourth exon of TNF-$\alpha$) are linked with one another simultaneously in their order.

An example of above 1) will be explained herebelow.

PHH58, which is a cDNA complementary to one of the mRNAs synthesized in the course of differentiation of malignant bone marrow into monocytes by stimulation with exogenous differentiation inducing agents (Biochemical and Biophysical Research Communications, vol. 132, No. 1, 100-109, 1985), is treated with PstI followed by recovery of the produced DNA fragment of about 530 bp. Then the fragment is treated with HinfI to give a PstI/HinfI fragment of about 220 bp and a HinfI/PstI fragment of about 310 bp. The former PstI/HinfI fragment is cleaved with Mn$\ell$I (Bairabos Inc., New England in USA) to give a 127 bp Mn$\ell$I/HinfI fragment which is then linked with the above HinfI/PstI fragment to construct a Mn$\ell$I/PstI fragment encoding the amino acid sequence of TH$\beta_4$. The resulting construction is linked with the Mn$\ell$I/PstI fragment of plasmid vector pUC540 to give plasmid pUC540TH$\beta_4$ of about 3.5 kbp.

4

PUC540 is a product of cloning of EcoRI/BamHI fragment from plasmid pDR540 having a tac promotor into plasmid vector pUC8 at the EcoRI/BamHI cleavage site; the two plasmids are commercially available from Pharmacia. Then the pUC540TH$\beta_4$ is subjected to treatments involving linking With BamHI linker to provide a DNA encoding both the amino acid sequence of TH$\beta_4$ and the amino acid sequence Asp-Pro which becomes the linkage connecting the former sequence to x-(the amino acid sequence corresponding to the base sequence constructed by linking guanine with the beginning of the base sequence of the fourth exon of TNF-$\alpha$).

The DNA encoding x- (the amino acid sequence corresponding to the base sequence constructed by linking guanine with the beginning of the base sequence of the fourth exon of TNF-$\alpha$) may be synthesized chemically essentially according to the process described in Nucleic Acids Res., 10, 7439-7448, 1981, Biochemistry, 17, 1257-1267, 1978, or elsewhere. For example, pUC540$^{TNF}$21/22 (corres. to the case where x is a part of the linkage), pUC540$^{TNF}$69/70 (corres. to the case where x represents the 1) referred to above) and pUC540$^{TNF}$ 72/73 (corres. to the case where x represents the 2) referred to above) disclosed in Example 2 of Japanese patent application disclosure No. 62-282587 (Japanese patent application No. 61-169522), pUC540AMCT-1 (corres. to the case where x represents the 3) referred to above) disclosed in Example 3 of the same document as the above, and pUC540AM1 (corres. to the case where x represents the 4) referred to above) and pUC540AM2 (corres. to the case where x represents the 5) referred to above) disclosed in working examples given later may be treated with NcoI (Japan Gene Inc.), Mung Bean nuclease and PstI to provide the object DNAs encoding x- (the amino acid sequence corresponding to the base sequence constructed by linking guanine with the beginning of the base sequence of the fourth exon of TNF-$\alpha$). This is because in those plasmids the DNA fragments encoding x-(the amino acid sequence corresponding to the base sequence constructed by linking guanine with the beginning of the base sequence of the fourth exon of TNF-$\alpha$) is linked with the Tac promotor downstream the BamHI cleavage site, and immediately after the BamHI cleavage site there exists the starting codon ATG which is followed by an amino acid the first codon of which is G. An embodiment of the case where pUC540AM1 is used will be explained in detail in an working example given later.

(2) Plasmid pUCTH$\beta_4$/x-(the amino acid sequence corresponding to the base sequence constructed by linking guanine with the beginning of the base sequence of the fourth exon of TNF-$\alpha$)

In order to incorporate DNAs of the present invention into a vector DNA in an expressible manner, as is well known, they are incorporated downstream the Shine-Dalgarno sequence (hereunder referred to as the SD sequence) of a vector DNA having a promotor sequence (being usually downstream of an operator sequence) and the SD sequence which is located downstream the promotor. Alternatively, first the DNAs of the present invention may be incorporated into a vector DNA and then a promotor sequence (usually together with an operator sequence) and the SD sequence are inserted upstream the DNAs.

Processes for expression of genetic information of an exogenous gene by techniques using recombinant DNAs are described generally in "Techniques for utilizing gene recombinant (4)", 1983, Science Forum; "Molecular Cloning", 1982, Cold Spring Harbor Lab.; "Introduction into cells and expression of recombinant genes", 1983, Kyoritsu Shuppan Cor.; etc.

The case where E. coli is used as the host will be illustrated in a working example given later.

Alternatively, in case yeast is used as the host, the genetic information of the DNAs of the present invention can be expressed as described hereunder.

Plasmid vector pMA56 with a promotor for alcohol dehydrogenase (ADHI) incorporated therein ("Nature", 298, pp. 347-350, 1982) has an EcoRI site downstream the promotor. Thus, the DNA of the present invention may be recovered as BamHI/PstI fragment from, for example, recombinants described in working examples given later and then may be insereted into pMA56 at the EcoRI site downstream the ADHI promotor thereof using EcoRI/BamHI linker and PstI/EcoRI linker thereby being controlled by the ADHI to express the genetic information in yeast.

Further, as repressible acidic phosphatase (PHO5) promotor-having pAM82 ("Proc. Natl. Acad. Sci. U.S.A.", 80, pp. 1-5, 1983) has an XhoI site downstream the PHO5 promotor, the DNAs of the present invention may be recovered as a BamHI/PstI fragment from, for example, recombinants described in working examples given later and then may be insereted into pMA82 at the XhoI site downstream the PH05 promotor thereof using BamHI/XhoI linker and PstI/XhoI linker thereby being controlled by the PHO5 promotor to express the genetic information in yeast.

B. subtilis may also be employed as the host as follows.

PTUB285 having $\alpha$-amylase promotor which is originally possessed by B. subtilis Marburs strain ("Gene", 34, p. 148, 1985) has a HincII site downstream the promotor and a signal peptide. Thus, the DNA of the present invention may be recovered as BamHI/PstI fragment from, for example, recombinants described in working examples given later , and then may be insereted into pTUB285 at its HincII site using HincII/BamHI linker and HincII/PstI linker which fits the amino acid frame of the signal peptide thereby being controlled by the α-amylase promotor to express the genetic information in B.

(3) Separation and purification of anti-tumor polypeptides

The host cells are collected by, for example, centrifugation, and then crushed by treatment with ultrasonic waves or lysozyme. Here a hypotonic solution is used, and in some cases coexistence of a surfactant such as SDS or guanidine HCℓ may produce a better result.

The crushed cell-containing solution is subjected to centrifugation to provide a supernatant.

The thus-prepared supernatant containing the anti-tumor polypeptide may be purified according to any conventional method of purifying proteins.

That is, the supernatant may be subjected to purification by ion exchange chromatography using a basic anion exchanger, salting out, dialysis, gel filtration, hydrophobic chromatography, high performance molecular sieve chromatography, electrophoresis, etc., in the given or any optional order or by any desired combination of these methods.

For example, the basic anion exchanger is preferred to be DEAE-Sephadex A-25 or A-50, Sepharose CL-6B, or DEAE-Sephamil (all made by Pharmacia AB), but any other diethylamino, aminoethyl, or quarternary-aminoethyl group-containing anion exchanger may be used.

Preferable embodiments of the buffer solution available for use include Tris-HCℓ and phosphate buffer solutions at pH 6.6-9.0. Any of these buffer solutions may be used at a low concentration of about 0.05 M to dilute the culture containing the anti-tumor polypeptide to a saline concentration of 0.1 M or less, and then the resulting solution is contacted with an anion exchanger which adsorbs the anti-tumor polypeptide.

The elution of the anti-tumor polypeptide is carried out with a saline solution containing 0.1-0.2 M of NaCℓ or KCℓ. The anti-tumor polypeptide is eluted at a saline concentration of about 0.2M. The contact with the anion exchanger is preferably effected by a column process, but a batch process may be employed if the contact is effected on a large scale.

In advance of the anion exchange chromatography, the solution is preferably pre-treated with an ultrafiltration membrane for removal of lower molecular materials, thereby improving the purification efficiency.

The solution resulting from the anion exchange chromatography is subjected to dialysis and concentration followed by gel filtration. Embodiments of carriers for the gel filtration include Sephadex G-75 and G-100 (manufactured by Pharmacia AB), Sephacryl S-200 (manufactured by Pharmacia AB), Biogel P-100 (manufactured by Biorad), and Toyo Pearl HW-50 and HW-55 (manufactured by Toyo Soda Corp.).

The buffer solution intended for use in the gel filtration may be a Tris-HCℓ or phosphate buffer solution at pH 6.0 - 9.0. To prevent adsorption it is desired that 0.2-0.5 M of a saline such as NaCℓ be added to the solution.

Alternatively, the anti-tumor polypeptide active solution may be purified by hydrophobic chromatography. Here, Butyl-Toyo Pearl 650 (manufactured by Toyo Soda Corp.) or the like may be used as the carrier, and a saline such as ammonium sulfate or NaCℓ is employed to elute the anti-tumor polypeptide.

The anti-tumor polypeptide-containing solution purified by gel filtration or hydrophobic chromatography is then subjected to fast protein exchange chromatography using a Pharmacia FPLC (Fast Protein, Peptide, Polynucleotide, Liquid Chromatography) system on a Mono Q HR5/5 column (high performance anion exchanger column manufactured by Pharmacia AB) to provide a purified sample.

The conditions for the fast protein anion exchange chromatography are the same as for the ion exchange chromatography using a carrier such as DESE-Sepharose mentioned above.

Roughly the novel DNAs of the present invention have three advantages.

The first avantage is in that they encode novel anti-tumor polypeptides. The anti-tumor properties are confirmed by their cytotoxicity to L-929 ("Proc. Natl. Acad. Sci. U.S.A.", 72, 3666-3670, 1983), Meth A fibrosarcoma ("Science", 230, 944, 1985) and others. In addition these novel polypeptides induce production of endogenous TNF to a high degree.

The second advantage is in that the polypeptides may be treated with an appropriate acid such as formic or acetic acid to cause their cleavage immediately before and behind the linkage Asp-Pro to give $TH\beta_4$ and the anti-tumor polypeptides: x-(the amino acid sequence corresponding to the base sequence constructed by linking guanine with the beginning of the base sequence of the fourth exon of TNF-α). As mentioned above, the DNAs of the present invention may be prepared on a large scale if they are incorporated into E. coli or the like. So, now the mass production of $TH\beta_4$ is practicable.

The third advantage is in that the DNAs of the present invention may be subjected to the action of restriction enzyme NruI (TCGCGA) to cleave the sequence just before that equivalent to the base sequence of the fourth exon of TNF-$\alpha$ to introduce any optional base sequence at the cleavage site in case the first amino acid alanine of the amino acid sequence corresponding to the base sequence constructed by linking guanine with the beginning of the base sequence of the fourth exon of TNF-$\alpha$ is represented by the base sequence GCG.

The cytotoxicity to L929 cells of the novel polypeptides encoded by the novel DNAs of the present invention may be analyzed as follows:

Cytotoxicity to L-929 cells

L-929 cells are cultured in Eagles' Minimum Essential Medium (hereunder referred to only as MEM) with 5 % fetal calf serum (hereunder referred to only as FCS) added thereto until 100 $\mu\ell$ of the medium contains $8 \times 10^4$ cells, and then the cells are grown in a flat-bottomed plate having 96 wells.

The growth conditions are 2 hours at 37°C in the presence of 5 % $CO_2$, and 100 % $H_2O$, and the procedures may be the same as for the conventional cell culture. Then actinomycin D is added to the medium to a final concentration of 1 $\mu$g/m$\ell$, and the volume of the culture solution is adjusted to 150 $\mu\ell$. Immediately thereafter 50 $\mu\ell$ of the sample diluted appropriately with MEM medium is added to the culture solution. Here, $ED_{50}$ may be determined by adjusting the dilution appropriately.

The L-929 cells having a final volume of 200 $\mu\ell$ are cultured for an additional 18 hours under the same conditions as described above.

In order to determine the cells necrosis activity, first the whole medium is removed followed by addition of a 2 % methyl alcoholic solution containing 0.2 % crystal violet for fixation staining. Crystal violet stains all the eukaryotic cells, but does not stain those cells left in the bottom of the flask as the result of necrosis; so the cell necrosis activity may be determined directly. The staining degree is measured on the basis of adsorption at $OD_{590\,nm}$, and is compared with that of a control to determine the cell necrosis activity. This activity is defined as follows.

The dilution of the sample which allows the survival of 50 % of L-929 cells (N) is determined. Rabbit TNS is used as the control, and its activity n (unit/m$\ell$) is determined using $2.4 \times 10^5$ units/mg/m$\ell$ of human TNF. The dilution which provides $ED_{50}$ of rabbit TNS is determined.

The activity of the sample (units/m$\ell$) is calculated by the equation N/C x n.

The cytotoxicity to Meth A fibrosarcoma, Lewis lung carcinoma (3LL), MH134 tumor and B16 melanoma may be determined substantially in the same manner as the above.

Hereunder, the present invention will be explained in more detail with reference to examples and experiments.

Example 1

(1) Preparation of DNA encoding (the amino acid sequence of TH$\beta_4$)-Asp-Pro-x-(the amino acid sequence corresponding to the base sequence constructed by linking guanine with the beginning of the base sequence of the fourth exon of TNF-$\alpha$)

1) One hundred $\mu$g of pHH58 was digested with 250 units of PstI at 37°C to separate off PstI/PstI fragment of 530 bp using 1.2% agarose gel. The fragment was extracted from the gel followed by purification of a hydroxy apatite column to recover 2.7 $\mu$g of DNA.

2) Then the DNA was digested with 12 units of HinfI to effect the respective recovery of $^{5'}$PstI/HinfI$^{3'}$ fragment of 220 bp and $^{5'}$HinfI/PstI$^{3'}$ fragment of 310 bp using 8% acrylamide gel. The recovery was carried out in a conventional manner using a DEAE cellulose column after the extraction of the two fragments from the gel. Their detection based on UV absorption (OD260 nm) was supposed to be impossible to effect, so the qunatity of the two fragments recovered was estimated according to the following equation on the assuption that 100% was recovered.

Quantity of PstI/HinfI fragment recovered
= 2.7 x 220 / (310 + 220) = 1.1 $\mu$g
Quantity of HinfI/PstI fragment recovered
= 2.7 x 310 / (310 + 220) = 1.6 $\mu$g

3) The PstI/HinfI fragment was digested with 8 units of Mn$C\ell$, and a Mn$C\ell$I/HinfI fragment of 127 bp was separated and recovered using 8% acrylamide gel; the yield was about 100-150 ng.

4) Twenty six ng of the Mnℓl/Hinfl fragment was mixed with 65 ng of Hinfl/PstI fragment followed by addition of 87 units of T4DNA ligase, and the mixture was reacted at 16°C for 1 hour. Then the enzyme was inactivated at 70°C for 5 minutes followed by addition of 15 units of PstI and a two hour culture at 37°C to give about 90 ng of DNA.

5) The DNA from the above 4) was mixed with the BamHI/PstI fragment of pUC540 (26ng) in the ethanol (final concentration: 70%)in the presence of 100mM NaCℓ. The precipitate was recovered and dissolved in 5 μℓ of distilled water and then subjected to freeze-drying to about 0.5 μℓ to remove the ethanol and the distilled water. Thereafter 0.4 μℓ of 10 x ligase reaction buffer solution (10mM ATP, 660mM Tris-HCℓ (pH 7.6), 66mM magnesium chloride and 50mM dithiothreithol) and 35 units of T4DNA ligase were added to the residue followed by culture at 4°C overnight to provide about 3.5 kbp of pUC540TH$\beta_4$.

6) One hundred μg of pUC540TH$\beta_4$ was digested with 190 units of Hinfl at 37°C overnight, and 50 μg of the digestion product was mixed with Klenow fragment of DNA polymerase I (40 units) in the presence of 80 μM of deoxy ATP and 80 μM in deoxy TTP followed by culture at room temperature for 30 minutes.

7) The Klenov fragment mentioned above was inactivated by healing at 70°C for 5 minutes, 120 units of BamHI was added to the culture solution followed by culture at 37°C for 3 hours.

8) The BamHI and Hinfl cleavage sites were repaired to flat ends with A and T using 8% acryl gel and about 450 ng of 135 bp of DNA was separated and recovered using a DKAK cellulose column.

9) Twenty seven ng of the DNA from the above 8) was mixed with 1.6 ng of BamHI linker in the presence of 87 units of T4DNA ligase followed by culture at 4°C overnight to yield DNA encoding (the amino acid sequence of TH$\beta_4$)-Asp-Pro.

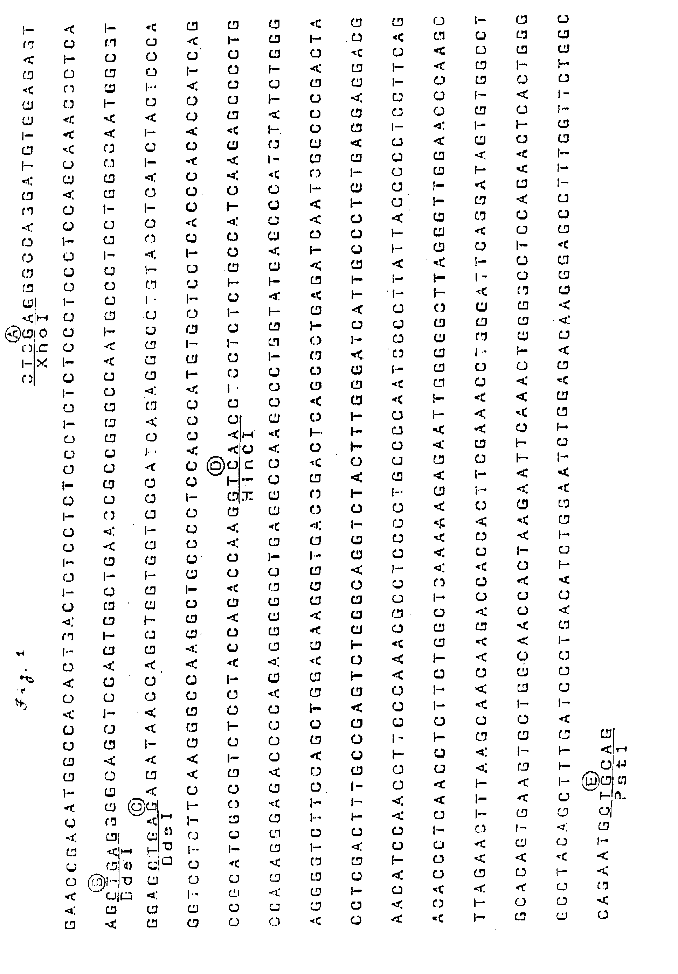

10) The genome gene of the anti-tumor polypeptide extracted from human acute monocytic leukemia cell THP-I (Japanese patent application disclosure No. 62-282587) was cleaved with XhoI and PstI to recover XhoI/PstI fragment shown in Fig. 1. Then this fragment was inserted into plasmid vector pUC540 having Tac promotor and SD sequence at the Saℓl/Pst cleavage site to prepare plasmid pUC540$^{TNF}$x/p.

11) Separately, the XhoI/PstI fragment shown in Fig. 1 was cleaved with HincII to recover 294 bp XHoI/HincII fragment and 521 bp HincII/PstI fragment. Further the 294 bp fragment was partially cleaved with DdcI to recover 206 bp DdeI/HincII fragment.

The thus-recovered 521 bp HincII/PstI fragment and 206 bp DdeI/HincII fragment were combined with 72/73 bp double-strand DNA synthesized with a DNA synthesizer 380A (ABI Corp. in USA) and shown in Table 1 given below followed by insertion into the pUC540$^{TNF}$x/p at the BamHI/PstI site to provide pUC540$^{TNF}$ AMI.

### Table 1

#### Base sequence of synthetic DNA (73/72 bp)

```
      MetValArgSerSerThrArgThrProSerArgLysProVal
      ┌─┐ ┌─┐ ┌─┐ ┌─┐ ┌─┐ ┌─┐ ┌─┐ ┌─┐ ┌─┐ ┌─┐ ┌─┐ ┌─┐ ┌─┐ ┌─┐
    5'-GATCCATGGTTAGAAGCTCCACCCGTACCCCGAGCCGTAAACCGGTA
       GTACCAATCTTCGAGGTGGGCATGGGGCTCGGCATTTGGCCAT

      AlaHisValValAlaAsnProGln
      ┌─┐ ┌─┐ ┌─┐ ┌─┐ ┌─┐ ┌─┐ ┌─┐ ┌─┐
      GCCCATGTTGTAGCAAACCCTCAAGC
      CGGGTACAACATCGTTTGGGAGTTCGACT
```

12) To 100 μg of pUC540$^{TNF}$AMI there was added 120 units of NcoI followed by culture at 37°C overnight. The completion of the digestion was confirmed by agar gel electrophoresis followed by extraction with phenol and then with chloroform and purification of the DNA on Sephadex G50 column.

13) To the DNA there was added 90 units of Mung Been nuclease and the mixture was allowed to stand at 37°C for 10 minutes. Then the mixture was heated at 70°C for 5 minutes to inactivate the nuclease.

14) The mixture was cultured at 37°C overnight after addition of 160 units of PstI. Then 5% acrylamide gel was used to recover 770 bp DNA (about 1 μg).

15) The DNA obtained by following the procedures described in the above 1) to 8) (28.6 ng) and the other DNA obtained at the end of procedures given in the above 9) to 14) (157 ng) were cultured at 4°C overnight together in the presence of 87 units of T4DNA ligase. Then the ligase was inactivated by heating at 70°C for 10 minutes.

16) A TWENTY hours culture was effected at 37°C after addition of 0.8 units of PstI, and the mixture was heated at 70°C for 10 minutes to inactivate the PstI. Then the mixture was mixed with the BamHI/PstI fragment of plasmid vector pUC540 (76 $\mu$g) followed by culture at 4°C overnight in the presence of 90 units of T4DNA ligase. Thus there was provided plasmid pUC540 (the amino acid sequence of TH$\beta_4$)-Asp-Pro-x-(the amino acid sequence corresponding to the base sequence constructed by linking guanine with the beginning of the base sequence of the fourth exon of TNF-$\alpha$) wherein x is Val-Arg-Ser-Ser-Thr-Arg-Thr-Pro-Ser-Arg-Lys-Pro-Val-Ala-His-Val-Val.

17) E. coli DH-1 with the plasmid from the above 16) incorporated therein was subjected to shaking culture at 37°C overnight in 10 m$\ell$ of NZY medium (prepared by mixing 5 g of NaC$\ell$, 2 g of MgC$\ell_2 \cdot 7H_2O$, 10 g of NZ amine A manufactured by Wako Jun-yaku in Japan and 5 g of yeast extracts in distilled water in a quanaity to prepare 1 $\ell$ of medium) containing 50 $\mu$g/m$\ell$ of ampicillin and 10 $\mu$g/m$\ell$ of kanamycin. Ten m$\ell$ of the resuting culture solution was added to 100 m$\ell$ of NZY medium containing 50 $\mu$g/m$\ell$ of ampicillin and 10 $\mu$g/m$\ell$ of kanamycin followed by shaking culture at 37°C for 6 hours. Then all the culture solution (about 100 m$\ell$) was added to 1 $\ell$ of NZY medium containing 0.7 mM of isopropyl $\beta$-D-galactopyranoside (hereunder referred to as IPTG). 50 $\mu$g/m$\ell$ of ampicillin and 10 $\mu$g/m$\ell$ of kanamycin followed by shaking culture at 37°C overnight.

18) The thus constructed E. coli was collected with a centrifuge (5000 rpm, 10 minutes) and suspended in 1 mM of phenylmethylsulfonyl chloride (a protease inhibitor). The resulting cell suspension was divided into 10 m$\ell$ portions to which ultrasonic waves were applied three times at graduation 40 of a Branson sonifier for 6 minutes to crush the E. coli cells.

19) The mixture was subjected to centrifugation at 100000 rpm for 1 hour to recover the supernatant. The TNF activity of this supernatant to L929 cells was $1.85/10^6$ units/m$\ell$.

20) Ammonium sulfate fractions (60% saturation) from the above supernatant were separated off and subjected to centrifugation at 10000 rpm for 30 minutes to provide precipitate which was then dissolved in 5 m$\ell$ of PBS(-) followed by dialysis against 5 $\ell$ of a solution containing 10 mM of Tris-HC$\ell$ (pH 7.4) and 10 mM of NaC$\ell$. Then the solution was treated by centrifugation at 13000 rpm overnight to remove the insolubles followed by recovery of the supernatant.

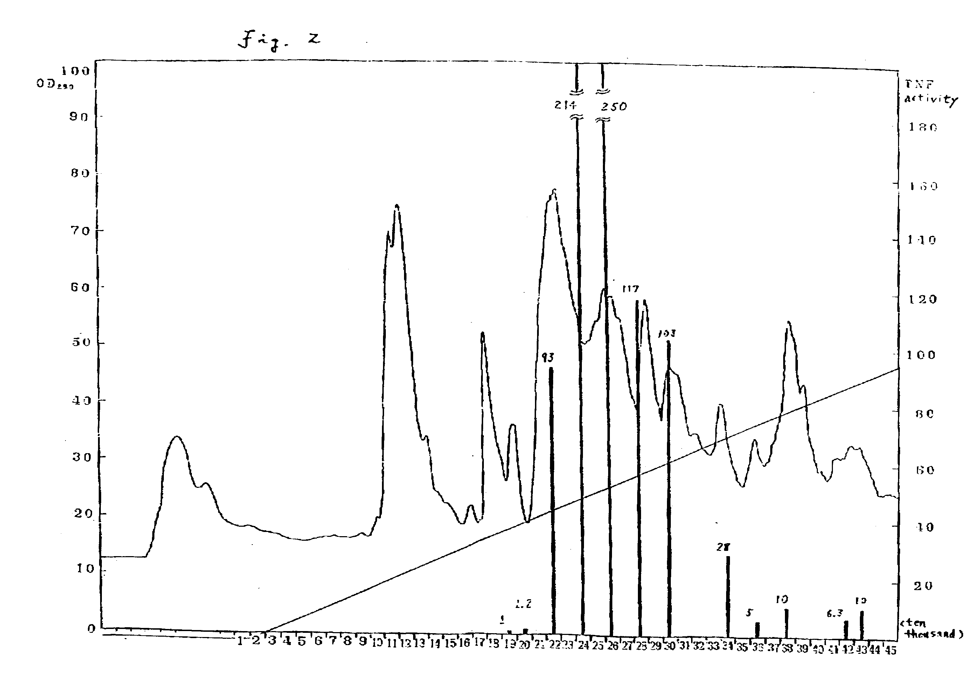

21) The supernatant was subjected to chromatography on Pharmacia Mono Q FPLC column. The flow rate was adjusted to 2.0 m$\ell$/min., and the 20 m$\ell$ portions were collected in a gradient elution manner wherein the NaC$\ell$ concentration was increased linearly from 0.01 M to 1 M. The elution pattern is shown in Fig. 2.

In Fig. 2, the consecutive numbers along the axis of abscissa represent the respective fractions, the curve traces the quantity of the proteins eluted, the columnar graph visualizes the TNF activity to L929 cells (units), and the linear shows the salt content of the eluates (0.01 M to 1 M).

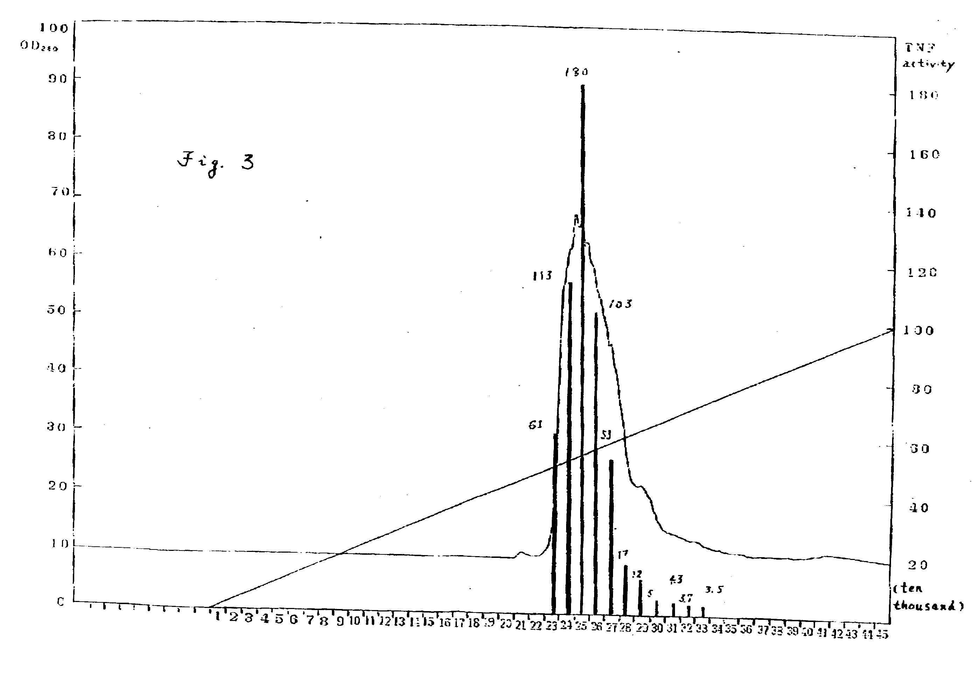

21) Some active fractions Nos. 23-27, which are rich in the protein having the structure: (the amino acid sequence of TH$\beta_4$)-Asp-Pro-x-(the amino acid sequence corresponding to the base sequence constructed by linking guanine with the beginning of the base sequence of the fourth exon of TNF-$\alpha$) wherein x is Val-Arg-Ser-Ser-Thr-Arg-Thr-Pro-Ser-Arg-Lys-Pro-Val-Ala- His-Val-Val (hereunder referred to as "chimera TNF"), were collected and subjected again to the treatment on a Mono Q column to provide 7.5 m$\ell$ of fraction showing only one peak (purity is over 95%). The elution pattern is shown in Fig. 3.

In Fig. 3, all the consecutive numbers along the axis of abscissa, the curve, the columnar graph and the linear are defined in the same manner as in Fig. 2.

The TNF activity of the above fraction to L929 cells was $2.5 \times 10^6$ units/m$\ell$. The study by gel filtration using a Pharmacia Superose 12 column revealed that the molecular weight is 67000 dalton or more, and suggested that the molecule is a trimer or tetramer. SDS electrophoresis showed that it is 22000 dalton. The LPS content was 12 ng/mg protein.

Reference 1

E. coli JM103 with the rDNA obtained by effecting the procedures described in 10) to 14) in Example 1 (hereunder referred to as rTNF-S-AM1) incorporated therein was pre-cultured in a 1 x YT medium (0.8% bactotrypton + 0.5% bactoyeast extracts + 0.5% NaC$\ell$) containing 50 $\mu$g/m$\ell$ of ampicillin at 37°C, and then transferred in a proportion of 1 % to a 500 m$\ell$ of a Sakaguchi flask containing 100 m$\ell$ of a 1 x YT medium with 50 $\mu$g/m$\ell$ of ampicillin added thereto. The mixture was cultured in the same manner at 37°C. When the $OD_{660}$ reached 0.3, IPTG was added to the culture to a final concentration of 0.7 mM followed by further culture for twenty-four hours. The thus-obtained E. coli was collected with a centrifuge, washed with 1 x PBS (0.8 % NaC$\ell$ + 0.02% KC$\ell$ + 0.02% KH$_2$PO$_4$ + 0.115% Na$_2$HPO$_4$), and suspended in 10 m$\ell$ of

1 x PBS, followed by application of ultrasonic waves to the culture to crush the E. coli cells. Then the polypeptide expressed by rTNF-S-AM1 was separated and purified in the conventional manner. This polypeptide (hereunder referred to as AM1) has an amino acid sequence shown below, and its molecular weight was 17500 ± 500 (SDS polyacrylamide electrophoresis).

Val-Arg-Ser-Ser-Thr-Arg-Thr-Pro-Ser-Arg-Lys-Pro-Val-Ala-

His-Val-Val

(the amino acid sequence corresponding to the base sequence constructed by linking guanine with the beginning of the base sequence of the fourth exon of TNF-α)

Reference 2

Following the procedures described in 10) to 16) of Example 1, but substituting Cys for the Ser which is the fifth amino acid of the synthetic DNA shown in Table 1 in Example 1, there was obtained another rDNA (hereunder referred to as rTNF-S-AM2). This recombinant was incorporated in E. coli JM103 and treated in the same manner as in Reference 1 to separate and purify the polypeptide expressed by rTNF-S-AM2. This polypeptide (hereunder referred to as AM2) has an amino acid sequence shown below, and its molecular weight was 17500 ± 500 (SDS polyacrylamide electrophoresis).

Val-Arg-Ser-Cys-Thr-Arg-Thr-Pro-Ser-Arg-Lys-Pro-Val-Ala-

His-Val-Val-

(the amino acid sequence corresponding to the base sequence constructed by linking guanine with the beginning of the base sequence of the fourth exon of TNF-α)

Experiment 1 Anti-tumor activity to Meth A tumor

A cell suspension of Meth A fibrosarcoma cells ($2 \times 10^5/50 \ \mu\ell$) was inoculated into 10-12 week old BALB/c male mice intradermally. Seven days later only mice having tumors of which diameter had attained about 5 to 8 mm were chosen for further therapeutic experiments.

The respective medicines tested were diluted with physiological saline containing 1 % of serum from normal mice, and 200 $\mu\ell$ solutions were administered to the respective animals intravenously. This administration was effected on the seventh, tenth and thirteenth day after the inoculation of the tumor cells. The tumor size was determined according to the equation:

$$\sqrt{a \times b} \ mm$$

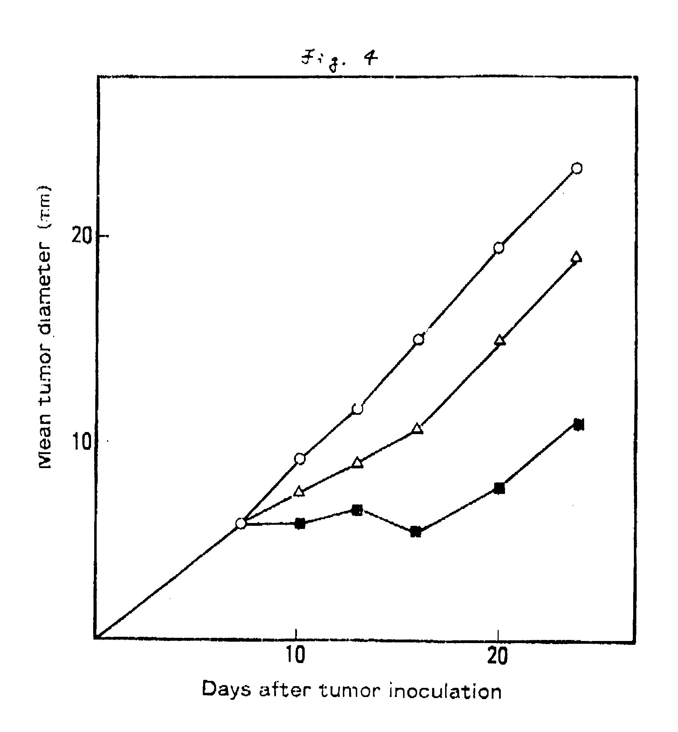

wherein
    "a"      denotes a shorter diameter and
    "b"      denotes a longer diameter.
The shorter and longer diameters were measured with slide calipers, and the results are shown in Fig. 4 as an average of 5.7 animals per group.

The administration of control (saline containing only 1 % serum from normal mice) failed to completely cure any one of the 7 animals tested. The data is shown in Fig. 4 by circles.

The administration of 10000 units of AM2 (LPS content : 910 pg/mg TNF protein) also failed to completely cure any one of the 5 animals tested. The data is shown in Fig. 4 by triangles.

The administration of 10000 units of chimera TNF succeeded in completely curing 2 of the 5 animals tested. The data is shown in Fig. 4 by filled squares.

10

Experiment 2 Anti-tumor activity in Lewis lung carcinoma (3LL)

A cell suspension of 3LL (3 x $10^5$/50 $\mu\ell$) was inoculated into 10-12 week old C57BL/6 male mice intradermally. Seven days later only mice having tumors of which diameter had attained about 5 to 8 mm were chosen for further therapeutic experiments.

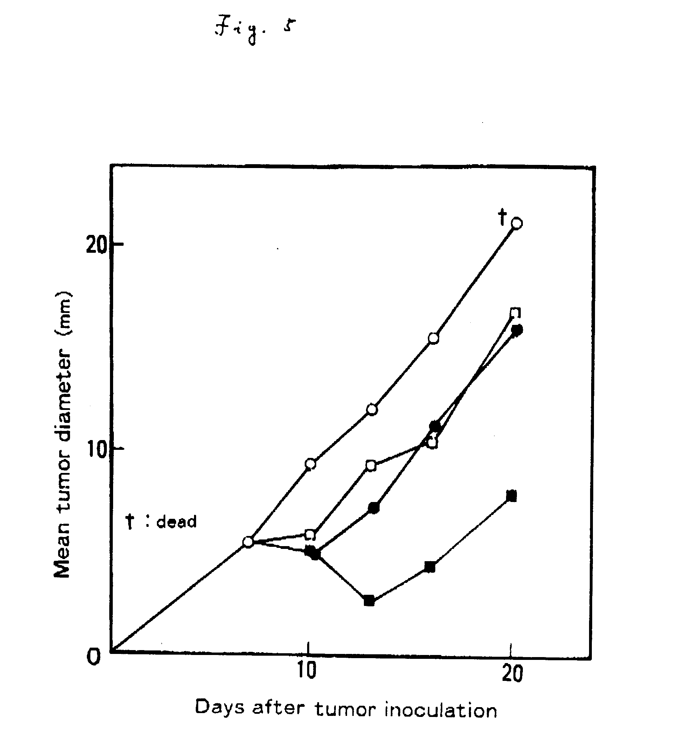

The respective medicines tested were administered to 5 to 6 mice per group only on the seventh or both on the seventh and thirteenth days after the inoculation of the tumor cells. The administration of control (saline containing only 1 % serum from normal mice) failed to completely cure any one of the 6 animals tested. The data is shown in Fig. 5 by empty circles.

The intravenous (i.v.) administration of 1 x $10^4$ units per 200 $\mu\ell$ of chimera TNF on both the seventh and the thirteenth days also failed to completely cure any one of the 5 animals tested. The data is shown in Fig. 5 by empty squares.

The intratumoral (i.t.) administration of 1 x $10^4$ units per 50 $\mu\ell$ of chimera TNF on the seventh day only succeeded in completely curing 3 of the 5 animals tested. The data is shown in Fig. 5 by filled squares.

The intratumoral (i.t.) administration of 1 x $10^5$ units per 50 $\mu\ell$ of AM1 (LPS content: 8.9 ng/mg TNF protein) on the seventh day only also failed to completely cure any one of the 5 animals tested. The data is shown in Fig. 5 by filled circles.

Experiment 3 Anti-tumor activity to MH134 hepatoma

A cell suspension of MH134 (2 x $10^5$/50 $\mu\ell$) was inoculated into 10-12 week old C3H/He male mice intradermally. Seven days later only mice having tumors of which diameter had attained about 6 to 9 mm were chosen for further therapeutic experiments.

The respective medicines tested were administered intravenously to 5 to 8 mice per group on the seventh, eleventh and fifteenth days after the inoculation of the tumor cells.

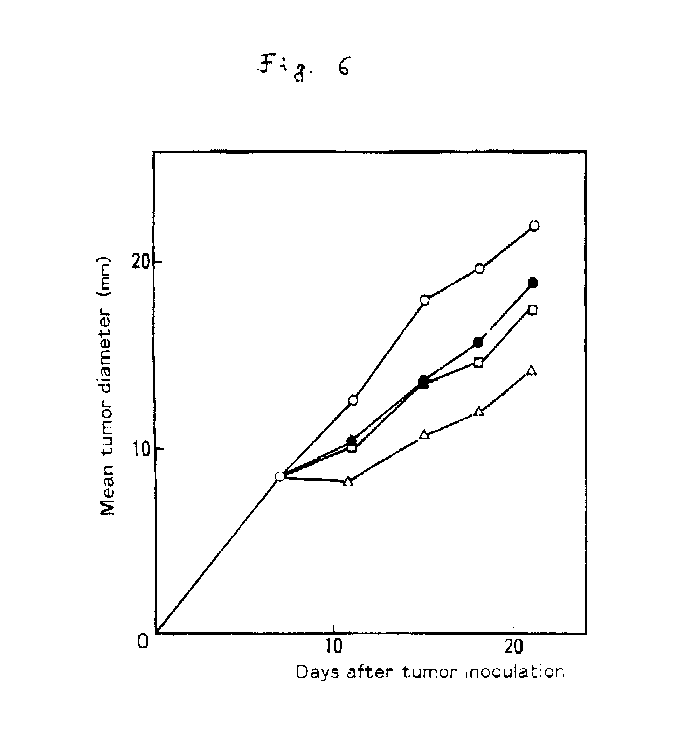

The administration of control (saline containing only 1 % serum from normal mice) failed to completely cure any one of the 8 animals tested. The data is shown in Fig. 6 by empty circles.

The administration of 3 x $10^3$ units per 200 $\mu\ell$ of chimera TNF also failed to completely cure any one of the 5 animals tested. The data is shown in Fig. 6 by empty squares.

The administration of 1 x $10^4$ units per 200 $\mu\ell$ of chimera TNF also failed to completely cure any one of the 5 animals tested. The data is shown in Fig. 6 by triangles.

The administration of 3 x $10^4$ units per 200 $\mu\ell$ of AM1 also failed to completely cure any one of the 5 animals tested. The data is shown in Fig. 6 by filled circlets.

Experiment 4 Anti-tumor activity to B16 melanoma

A cell suspension of B16 (1 x $10^6$/50 $\mu\ell$) was inoculated into 10-12 week old C57BL/6 male mice intradermally. Nine days later only mice having tumors of which diameter had attained about 5 to 9 mm were chosen for further therapeutic experiments.

The intravenous administration of the medicines tested was effected to 4 to 5 mice per group on the nineth, sixteenth and twenty-third days after the inoculation of the tumor cells.

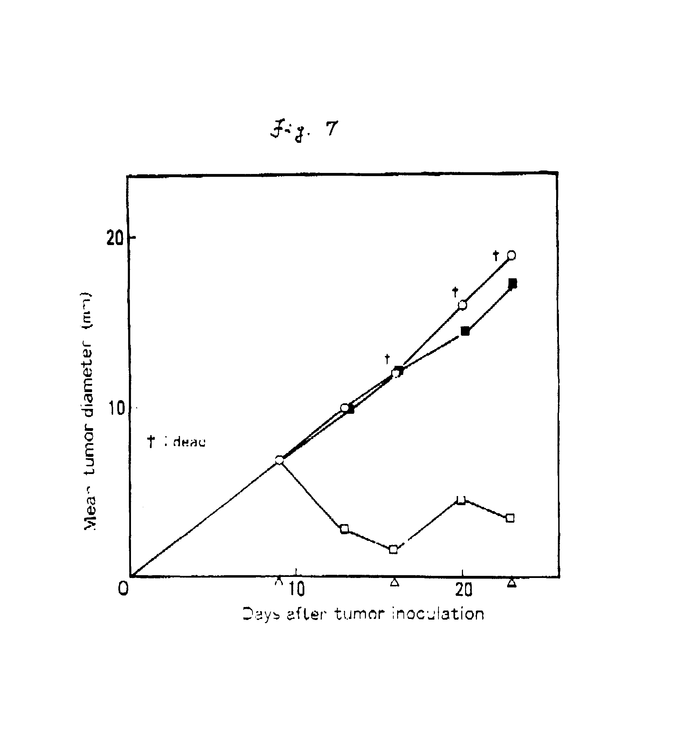

The administration of control (saline containing only 1 % serum from normal mice) failed to completely cure any one of the 5 animals tested. The data is shown in Fig. 7 by empty circlets.

The administration of 1 x $10^4$ units per 200 $\mu\ell$ of chimera TNF succeeded in completely curing 2 of the 4 animals tested. The data is shown in Fig. 7 by empty squares.

The administration of 1 x $10^4$ units per 200 $\mu\ell$ of AM1 failed to completely cure any one of the 4 animals tested. The data is shown in Fig. 7 by filled squares.

Experiment 5 Induction of production of endogenous TNF

One or 10000 units (based on the activity against L929) of chimera TNF obtained in Example 1 or AM1 obtained in Reference 1 was administered to 7 week old C3H/He male mice via caudal vein as a primer. Only physiological saline was given to the control.

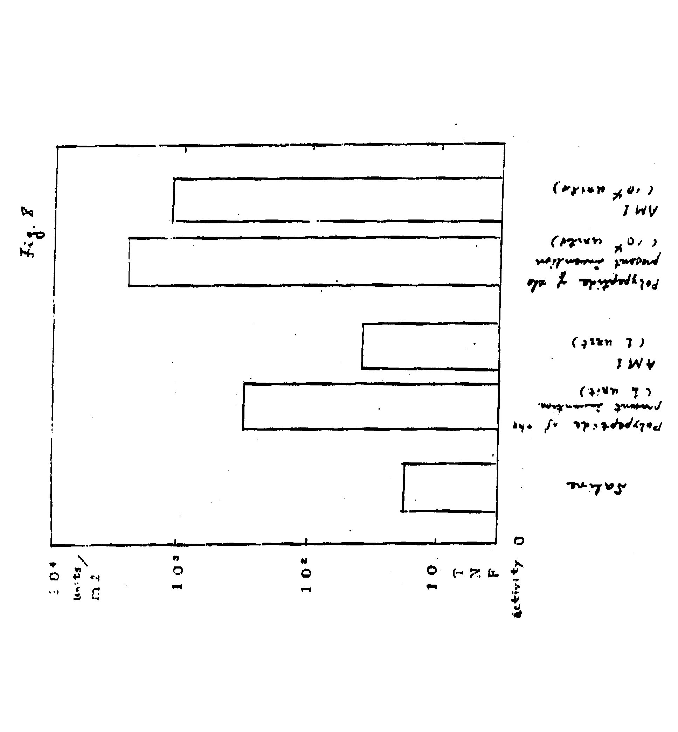

Three hours later 3 KE of OK-432 as a trigger was administered to the animals via caudal vein. Here "KE" is a Klinische Einheit unit, and 1 KE corresponds to the quantity of a preparation containing 0.1 mg of dried cells. Two hours later the blood was drawn from the inguinal artery, and the serum was separated to determine its TNF activity on the basis of the behavior against L929 cells. The results calculated as an average of three per group (an average of four in the case of AM1) are shown in Fig. 8.

11

EP 0 341 100 B1

As is shown in Fig. 8, only one unit of chimera TNF of the present invention improved the production of endogenous TNF by about twenty times, whereas the use of AM1 did not produce any effects. The two improved said production by 70-150 times when they are administered at a dosage of 10000 units.

Experiment 6

Chimera TNF obtained in Example 1 was freeze-dried, and was allowed to react in 70% formic acid at 37°C at a final concentration of 0.5-2 mg/ml. The resulting proteins were detected by SDS polyacrylamide electrophoresis under the following conditions:

| | |
|---|---|
| Separation gel: | 15 % acrylamide + 0.4 % bisacrylamide + 0.375 M Tris-HCl (pH 8.8) + 0.1 % SDS + 0.08 % TEMED (N, N, N, N-tetramethyleneethylenediamine + 0.08 % ammonium persulfate. |
| Concetration gel: | 5 % acrylamide + 0.13 % bisacrylamide + 0.125 M Tris-HCl (pH 6.8) + 0.1 % SDS + 0.2 % TEMED + 0.075 % ammonium persulfate. |
| Migration buffer: | 0.025 M Tris-HCl + 0.192 M glycine + 0.1 % SDS (pH about 8.3). |
| Migration conditions : | About 4.5 to 5 hours at a constant current of 20 mA |
| Dyeing and decoloring: | At the end of migration the gel is fixed with 20 % of trichloroacetic acid at room temperature for 30 minutes, and the protein bands are dyed with a 0.25 % solution of Coomassie brilliant blue R-250 in an ethanol- acetic acid- water (9:2:9) mixture. The decoloring is effected using an ethanol-acetic acid- water (25:8:65) mixture. |

As a result, there were detected bands indicating molecular weights of about 17000 and about 5000, respectively.

These bands were confirmed to combine with an antibody against TNF and one against TH$\beta_4$.

**Claims**
**Claims for the following Contracting States : AT, BE, CH, DE, FR, GB, GR, IT, LI, LU, NL, SE**

1.  DNA characterized by coding the following amino acid sequence:
    (The amino acid sequence of TH$\beta_4$)-Asp-Pro-x-(the amino acid sequence corresponding to the base sequence constructed by linking guanine with the beginning of the base sequence of the fourth exon of TNF-$\alpha$ )
    wherein X is a part of the linkage or represents any of the following amino acid sequences:

    1) Val-Ser-Ser-Ser-Arg-Thr-Pro-Ser-Asp-Lys-Pro-Val-Ala-His-Val-Val;

    2) Val-Arg-Ser-Ser-Ser-Arg-Thr-Pro-Ser-Asp-Lys-Pro-Val-Ala-His-Val-Val;

    3) Val-Lys-Ser-Cys-Thr-Arg-Thr-Pro-Ser-Arg-Lys-Pro-Val-Ala-His-Val-Val;

    4) Val-Arg-Ser-Ser-Thr-Arg-Thr-Pro-Ser-Arg-Lys-Pro-Val-Ala-His-Val-Val; and

    5) Val-Arg-Ser-Cys-Thr-Arg-Thr-Pro-Ser-Arg-Lys-Pro-Val-Ala-His-Val-Val.

2.  A process for preparing DNA which codes the following amino acid sequence:
    (The amino acid sequence of TH$\beta_4$)-Asp-Pro-x-(the amino acid sequence corresponding to the

12

EP 0 341 100 B1

base sequence constructed by linking guanine with the beginning of the base sequence of the fourth exon of TNF-$\alpha$ )

wherein X is a part of the linkage or represents any of the following amino acid sequences:

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; and

5) Val-Arg-Ser-Cys-Thr-Arg-Thr-Pro-Ser-Arg-Lys-Pro-Val-Ala-His-Val-Val.

characterized by comprising any one of the following steps:

1) DNA coding (the amino acid sequence of TH$\beta_4$)-Asp-Pro is linked with DNA coding x-(the amino acid sequence corresponding to the base sequence constructed by linking guanine with the beginning of the base sequence of the fourth exon of TNF-$\alpha$);

2) DNA coding (the amino acid sequence of TH$\beta_4$) is linked with DNA coding Asp-Pro-x-(the amino acid sequence corresponding to the base sequence constructed by linking guanine with the beginning of the base sequence of the fourth exon of TNF-$\alpha$); or

3) DNA coding (the amino acid sequence of TH$\beta_4$), DNA coding Asp-Pro and DNA coding x-(the amino acid sequence corresponding to the base sequence constructed by linking guanine with the beginning of the base sequence of the fourth exon of TNF-$\alpha$) are linked with one another simultaneously in their order.

3. Plasmid which incorporates therein DNA coding the following amino acid sequence:.

(The amino acid sequence of TH$\beta_4$)-Asp-Pro-x-(the amino acid sequence corresponding to the base sequence constructed by linking guanine with the beginning of the base sequence of the fourth exon of TNF-$\alpha$ )

wherein X is a part of the linkage or represents any of the following amino acid sequences:

13

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; and

5) Val-Arg-Ser-Cys-Thr-Arg-Thr-Pro-Ser-Arg-Lys-Pro-Val-Ala-His-Val-Val.

4. Polypeptide represented by the following amino acid sequence:

(The amino acid sequence of TH$\beta_4$)-Asp-Pro-x-(the amino acid sequence corresponding to the base sequence constructed by linking guanine with the beginning of the base sequence of the fourth exon of TNF-$\alpha$ )

wherein X is a part of the linkage or represents any of the following amino acid sequences:

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; and

5) Val-Arg-Ser-Cys-Thr-Arg-Thr-Pro-Ser-Arg-Lys-Pro-Val-Ala-His-Val-Val.

5. A process for preparing a polypeptide represented by the following amino acid sequence:

(The amino acid sequence of TH$\beta_4$)-Asp-Pro-x-(the amino acid sequence corresponding to the base sequence constructed by linking guanine with the beginning of the base sequence of the fourth exon of TNF-$\alpha$ )

wherein X is a part of the linkage or represents any of the following amino acid sequences:

14

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; and

5) Val-Arg-Ser-Cys-Thr-Arg-Thr-Pro-Ser-Arg-Lys-Pro-Val-Ala-His-Val-Val.

characterized by culturing a microorganism which incorporates therein a plasmid capable of growing in the host microorganism and incorporating therein DNA which codes the above amino acid sequence.

6. An anti-tumor agent comprising a polypeptide represented by the following amino acid sequence:
(The amino acid sequence of TH$\beta_4$)-Asp-Pro-x-(the amino acid sequence corresponding to the base sequence constructed by linking guanine with the beginning of the base sequence of the fourth exon of TNF-$\alpha$ )
wherein X is a part of the linkage or represents any of the following amino acid sequences:

1) Val-Ser-Ser-Ser-Arg-Thr-Pro-Ser-Asp-Lys-Pro-Val-Ala-

His-Val-Val;

2) Val-Arg-Ser-Ser-Ser-Arg-Thr-Pro-Ser-Asp-Lys-Pro-Val-

Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; and

5) Val-Arg-Ser-Cys-Thr-Arg-Thr-Pro-Ser-Arg-Lys-Pro-Val-Ala-His-Val-Val.

**Claims for the following Contracting State : ES**

1. A process for preparing DNA which encodes the following amino acid sequence:
(The amino acid sequence of TH$\beta_4$)-Asp-Pro-x-(the amino acid sequence corresponding to the base sequence constructed by linking guanine with the beginning of the base sequence of the fourth

EP 0 341 100 B1

exon of TNF-α )

wherein X is a part of the linkage or represents any of the following amino acid sequences:

```
1) Val-Ser-Ser-Ser-Arg-Thr-Pro-Ser-Asp-Lys-Pro-Val-Ala-
   His-Val-Val;

2) Val-Arg-Ser-Ser-Ser-Arg-Thr-Pro-Ser-Asp-Lys-Pro-Val-
   Ala-His-Val-Val;

3) Val-Lys-Ser-Cys-Thr-Arg-Thr-Pro-Ser-Arg-Lys-Pro-Val-
   Ala-His-Val-Val;

4) Val-Arg-Ser-Ser-Thr-Arg-Thr-Pro-Ser-Arg-Lys-Pro-Val-
   Ala-His-Val-Val;  and

5) Val-Arg-Ser-Cys-Thr-Arg-Thr-Pro-Ser-Arg-Lys-Pro-Val-
   Ala-His-Val-Val.
```

characterized by comprising any one of the following steps:

1) DNA encoding (the amino acid sequence of TH$\beta_4$)-Asp-Pro is linked with DNA encoding x-(the amino acid sequence corresponding to the base sequence constructed by linking guanine with the beginning of the base sequence of the fourth exon of TNF-α);

2) DNA encoding (the amino acid sequence of TH$\beta_4$) is linked with DNA encoding Asp-Pro-x-(the amino acid sequence corresponding to the base sequence constructed by linking guanine with the beginning of the base sequence of the fourth exon of TNF-α); or

3) DNA encoding (the amino acid sequence of TH$\beta_4$), DNA encoding Asp-Pro and DNA encoding x-(the amino acid sequence corresponding to the base sequence constructed by linking guanine with the beginning of the base sequence of the fourth exon of TNF-α) are linked with one another simultaneously in their order.

2.  A process for preparing a plasmid containing a DNA encoding the following amino acid sequence:.

(The amino acid sequence of TH$\beta_4$)-Asp-Pro-x-(the amino acid sequence corresponding to the base sequence constructed by linking guanine with the beginning of the base sequence of the fourth exon of TNF-α )

wherein X is a part of the linkage or represents any of the following amino acid sequences:

16

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; and

5) Val-Arg-Ser-Cys-Thr-Arg-Thr-Pro-Ser-Arg-Lys-Pro-Val-Ala-His-Val-Val.

wherein said DNA is incorporated into a vector DNA with a promotor

3. A process for preparing a polypeptide represented by the following amino acid sequence:
(The amino acid sequence of TH$\beta_4$)-Asp-Pro-x-(the amino acid sequence corresponding to the base sequence constructed by linking guanine with the beginning of the base sequence of the fourth exon of TNF-$\alpha$ )
wherein X is a part of the linkage or represents any of the following amino acid sequences:

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; and

5) Val-Arg-Ser-Cys-Thr-Arg-Thr-Pro-Ser-Arg-Lys-Pro-Val-Ala-His-Val-Val.

characterized by culturing a microorganism which incorporates therein a plasmid capable of growing in the host microorganism and incorporating therein DNA which encodes the above amino acid sequence.

4. A process for preparing an anti-tumor agent wherein a polypeptide represented by the following amino acid sequence:
(The amino acid sequence of TH$\beta_4$)-Asp-Pro-x-(the amino acid sequence corresponding to the base sequence constructed by linking guanine with the beginning of the base sequence of the fourth

exon of TNF-α )

wherein X is a part of the linkage or represents any of the following amino acid sequences:

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; and

5) Val-Arg-Ser-Cys-Thr-Arg-Thr-Pro-Ser-Arg-Lys-Pro-Val-Ala-His-Val-Val.

is admixed with an acceptable carrier.

**Patentansprüche**
**Patentansprüche für folgende Vertragsstaaten : AT, BE, CH, DE, FR, GB, GR, IT, LI, LU, NL, SE**

1. DNA, dadurch gekennzeichnet, daß sie die folgende Aminosäuresequenz codiert:
(Aminosäuresequenz von TH$\beta_4$)-Asp-Pro-x-(Aminosäuresequenz entsprechend der Basensequenz, hergestellt durch Verbinden von Guanin mit dem Beginn der Basensequenz des vierten Exons von TNF-α),
worin X einen Teil der Bindung oder eine der folgenden Aminosäuresequenzen darstellt:

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; und

5) Val-Arg-Ser-Cys-Thr-Arg-Thr-Pro-Ser-Arg-Lys-Pro-Val-Ala-His-Val-Val.

**2.** Verfahren zur Herstellung von DNA, welche die folgende Aminosäuresequenz codiert:

(Aminosäuresequenz von TH$\beta_4$)-Asp-Pro-x-(Aminosäuresequenz entsprechend der Basensequenz, hergestellt durch Verbinden von Guanin mit dem Beginn der Basensequenz des vierten Exons von TNF-$\alpha$),

worin X einen Teil der Bindung oder eine der folgenden Aminosäuresequenzen darstellt:

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; und

5) Val-Arg-Ser-Cys-Thr-Arg-Thr-Pro-Ser-Arg-Lys-Pro-Val-Ala-His-Val-Val

dadurch gekennzeichnet, daß es eine der folgenden Stufen umfaßt:

1) DNA, die (Aminosäuresequenz von TH$\beta_4$)-Asp-Pro codiert, wird mit DNA, die x-(Aminosäuresequenz entsprechend der Basensequenz, hergestellt durch Verbinden von Guanin mit dem Beginn der Basensequenz des vierten Exons von TNF-$\alpha$) codiert, verbunden;

2) DNA, die (Aminosäuresequenz von TH$\beta_4$) codiert, wird mit DNA, die Asp-Pro-x-(Aminosäuresequenz entsprechend der Basensequenz, hergestellt durch Verbinden von Guanin mit dem Beginn der Basensequenz des vierten Exons von TNF-$\alpha$) codiert, verbunden; oder

3) DNA, die (Aminosäuresequenz von TH$\beta_4$) codiert, DNA, die Asp-Pro codiert, und DNA, die x-(Aminosäuresequenz entsprechend der Basensequenz, hergestellt durch Verbinden von Guanin mit dem Beginn der Basensequenz des vierten Exons von TNF-$\alpha$) codiert, werden in der genannten Reihenfolge gleichzeitig miteinander verbunden.

**3.** Plasmid, das DNA enthält, welche die folgende Aminosäuresequenz codiert:

(Aminosäuresequenz von TH$\beta_4$)-Asp-Pro-x-(Aminosäuresequenz entsprechend der Basensequenz, hergestellt durch Verbinden von Guanin mit dem Beginn der Basensequenz des vierten Exons von TNF-$\alpha$),

worin X einen Teil der Bindung oder eine der folgenden Aminosäuresequenzen darstellt:

19

```
1) Val-Ser-Ser-Ser-Arg-Thr-Pro-Ser-Asp-Lys-Pro-Val-Ala-
   His-Val-Val;

2) Val-Arg-Ser-Ser-Ser-Arg-Thr-Pro-Ser-Asp-Lys-Pro-Val-
   Ala-His-Val-Val;

3) Val-Lys-Ser-Cys-Thr-Arg-Thr-Pro-Ser-Arg-Lys-Pro-Val-
   .Ala-His-Val-Val;

4) Val-Arg-Ser-Ser-Thr-Arg-Thr-Pro-Ser-Arg-Lys-Pro-Val-
   Ala-His-Val-Val; und

5) Val-Arg-Ser-Cys-Thr-Arg-Thr-Pro-Ser-Arg-Lys-Pro-Val-
   Ala-His-Val-Val.
```

**4.** Polypeptid, dargestellt durch die folgende Aminosäuresequenz:
(Aminosäuresequenz von $TH\beta_4$)-Asp-Pro-x-(Aminosäuresequenz entsprechend der Basensequenz, hergestellt durch Verbinden von Guanin mit dem Beginn der Basensequenz des vierten Exons von TNF-$\alpha$),
worin X einen Teil der Bindung oder eine der folgenden Aminosäuresequenzen darstellt:

```
1) Val-Ser-Ser-Ser-Arg-Thr-Pro-Ser-Asp-Lys-Pro-Val-Ala-
   His-Val-Val;

2) Val-Arg-Ser-Ser-Ser-Arg-Thr-Pro-Ser-Asp-Lys-Pro-Val-
   Ala-His-Val-Val;

3) Val-Lys-Ser-Cys-Thr-Arg-Thr-Pro-Ser-Arg-Lys-Pro-Val-
   .Ala-His-Val-Val;

4) Val-Arg-Ser-Ser-Thr-Arg-Thr-Pro-Ser-Arg-Lys-Pro-Val-
   Ala-His-Val-Val; und

5) Val-Arg-Ser-Cys-Thr-Arg-Thr-Pro-Ser-Arg-Lys-Pro-Val-
   Ala-His-Val-Val.
```

**5.** Verfahren zur Herstellung eines Polypeptids, dargestellt durch die folgende Aminosäuresequenz:
(Aminosäuresequenz von $TH\beta_4$)-Asp-Pro-x-(Aminosäuresequenz entsprechend der Basensequenz, hergestellt durch Verbinden von Guanin mit dem Beginn der Basensequenz des vierten Exons von TNF-$\alpha$),
worin X einen Teil der Bindung oder eine der folgenden Aminosäuresequenzen darstellt:

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; und

5) Val-Arg-Ser-Cys-Thr-Arg-Thr-Pro-Ser-Arg-Lys-Pro-Val-Ala-His-Val-VaL

dadurch gekennzeichnet, daß ein Mikroorganismus kultiviert (gezüchtet) wird, der ein Plasmid enthält, das in dem Wirts-Mikroorganismus wachsen kann und das DNA enthält, welche die obengenannte Aminosäuresequenz codiert.

6. Antitumor-Agens, das ein Polypeptid mit der nachstehend angegebenen Aminosäuresequenz umfaßt (enthält):
(Aminosäuresequenz von TH$\beta_4$)-Asp-Pro-x-(Aminosäuresequenz entsprechend der Basensequenz, hergestellt durch Verbinden von Guanin mit dem Beginn der Basensequenz des vierten Exons von TNF-$\alpha$),
worin X einen Teil der Bindung oder eine der folgenden Aminosäuresequenzen darstellt:

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; und

5) Val-Arg-Ser-Cys-Thr-Arg-Thr-Pro-Ser-Arg-Lys-Pro-Val-Ala-His-Val-VaL.

21

EP 0 341 100 B1

**Patentansprüche für folgenden Vertragsstaat : ES**

1. Verfahren zur Herstellung von DNA, welche die folgende Aminosäuresequenz codiert:
(Aminosäuresequenz von TH$\beta_4$)-Asp-Pro-x-(Aminosäuresequenz entsprechend der Basensequenz, hergestellt durch Verbinden von Guanin mit dem Beginn der Basensequenz des vierten Exons von TNF-$\alpha$),
worin X einen Teil der Bindung oder eine der folgenden Aminosäuresequenzen darstellt:

    1) Val-Ser-Ser-Ser-Arg-Thr-Pro-Ser-Asp-Lys-Pro-Val-Ala-His-Val-Val;

    2) Val-Arg-Ser-Ser-Ser-Arg-Thr-Pro-Ser-Asp-Lys-Pro-Val-Ala-His-Val-Val;

    3) Val-Lys-Ser-Cys-Thr-Arg-Thr-Pro-Ser-Arg-Lys-Pro-Val-Ala-His-Val-Val;

    4) Val-Arg-Ser-Ser-Thr-Arg-Thr-Pro-Ser-Arg-Lys-Pro-Val-Ala-His-Val-Val; und

    5) Val-Arg-Ser-Cys-Thr-Arg-Thr-Pro-Ser-Arg-Lys-Pro-Val-Ala-His-Val-Val

dadurch gekennzeichnet, daß es eine der folgenden Stufen umfaßt:
1) DNA, die (Aminosäuresequenz von TH$\beta_4$)-Asp-Pro codiert, wird mit DNA, die x-(Aminosäuresequenz entsprechend der Basensequenz, hergestellt durch Verbinden von Guanin mit dem Beginn der Basensequenz des vierten Exons von TNF-$\alpha$) codiert, verbunden;
2) DNA, die (Aminosäuresequenz von TH$\beta_4$) codiert, wird mit DNA, die Asp-Pro-x-(Aminosäuresequenz entsprechend der Basensequenz, hergestellt durch Verbinden von Guanin mit dem Beginn der Basensequenz des vierten Exons von TNF-$\alpha$) codiert, verbunden; oder
3) DNA, die (Aminosäuresequenz von TH$\beta_4$) codiert, DNA, die Asp-Pro codiert, und DNA, die x-(Aminosäuresequenz entsprechend der Basensequenz, hergestellt durch Verbinden von Guanin mit dem Beginn der Basensequenz des vierten Exons von TNF-$\alpha$) codiert, werden in der genannten Reihenfolge gleichzeitig miteinander verbunden.

2. Verfahren zur Herstellung eines Plasmids, das DNA enthält, welche die folgende Aminosäuresequenz codiert:
(Aminosäuresequenz von TH$\beta_4$)-Asp-Pro-x-(Aminosäuresequenz entsprechend der Basensequenz, hergestellt durch Verbinden von Guanin mit dem Beginn der Basensequenz des vierten Exons von TNF-$\alpha$),
worin X einen Teil der Bindung oder eine der folgenden Aminosäuresequenzen darstellt:

22

```
1) Val-Ser-Ser-Ser-Arg-Thr-Pro-Ser-Asp-Lys-Pro-Val-Ala-
   His-Val-Val;

2) Val-Arg-Ser-Ser-Ser-Arg-Thr-Pro-Ser-Asp-Lys-Pro-Val-
   Ala-His-Val-Val;

3) Val-Lys-Ser-Cys-Thr-Arg-Thr-Pro-Ser-Arg-Lys-Pro-Val-
   Ala-His-Val-Val;



4) Val-Arg-Ser-Ser-Thr-Arg-Thr-Pro-Ser-Arg-Lys-Pro-Val-
   Ala-His-Val-Val;  und

5) Val-Arg-Ser-Cys-Thr-Arg-Thr-Pro-Ser-Arg-Lys-Pro-Val-
   Ala-His-Val-Val
```

worin die genannte DNA einer Vektor-DNA mit einem Promotor einverleibt wird.

3. Verfahren zur Herstellung eines Polypeptids, dargestellt durch die folgende Aminosäuresequenz: (Aminosäuresequenz von TH$\beta_4$)-Asp-Pro-x-(Aminosäuresequenz entsprechend der Basensequenz, hergestellt durch Verbinden von Guanin mit dem Beginn der Basensequenz des vierten Exons von TNF-$\alpha$),
worin X einen Teil der Bindung oder eine der folgenden Aminosäuresequenzen darstellt:

```
1) Val-Ser-Ser-Ser-Arg-Thr-Pro-Ser-Asp-Lys-Pro-Val-Ala-
   His-Val-Val;

2) Val-Arg-Ser-Ser-Ser-Arg-Thr-Pro-Ser-Asp-Lys-Pro-Val-
   Ala-His-Val-Val;

3) Val-Lys-Ser-Cys-Thr-Arg-Thr-Pro-Ser-Arg-Lys-Pro-Val-
   Ala-His-Val-Val;

4) Val-Arg-Ser-Ser-Thr-Arg-Thr-Pro-Ser-Arg-Lys-Pro-Val-
   Ala-His-Val-Val; und

5) Val-Arg-Ser-Cys-Thr-Arg-Thr-Pro-Ser-Arg-Lys-Pro-Val-
   Ala-His-Val-Val
```

dadurch gekennzeichnet, daß ein Mikroorganismus kultiviert (gezüchtet) wird, der ein Plasmid enthält, das in dem Wirts-Mikroorganismus wachsen kann und DNA enthält, welche die obengenannte Aminosäuresequenz codiert.

**4.** Verfahren zur Herstellung eines Antitumor-Agens, bei dem ein Polypeptid, dargestellt durch die folgende Aminosäuresequenz:
(Aminosäuresequenz von TH$\beta_4$)-Asp-Pro-x-(Aminosäuresequenz entsprechend der Basensequenz, hergestellt durch Verbinden von Guanin mit dem Beginn der Basensequenz des vierten Exons von TNF-$\alpha$),
worin X einen Teil der Bindung oder eine der folgenden Aminosäuresequenzen darstellt:

```
1) Val-Ser-Ser-Ser-Arg-Thr-Pro-Ser-Asp-Lys-Pro-Val-Ala-
   His-Val-Val;

2) Val-Arg-Ser-Ser-Ser-Arg-Thr-Pro-Ser-Asp-Lys-Pro-Val-
   Ala-His-Val-Val;

3) Val-Lys-Ser-Cys-Thr-Arg-Thr-Pro-Ser-Arg-Lys-Pro-Val-
   Ala-His-Val-Val;

4) Val-Arg-Ser-Ser-Thr-Arg-Thr-Pro-Ser-Arg-Lys-Pro-Val-
   Ala-His-Val-Val; und

5) Val-Arg-Ser-Cys-Thr-Arg-Thr-Pro-Ser-Arg-Lys-Pro-Val-
   Ala-His-Val-Val
```

mit einem akzeptablen Träger gemischt wird.

**Revendications**
**Revendications pour les Etats contractants suivants : AT, BE, CH, DE, FR, GB, GR, IT, LI, LU, NL, SE**

**1.** ADN caractérisé en ce qu'il code pour la séquence d'acides aminés suivante:
(la séquence d'acides aminés de TH$\beta_4$)-Asp-Pro-x-(la séquence d'acides aminés correspondant à la séquence de bases construite en liant la guanine avec le début de la séquence de bases du quatrième exon de TNF-$\alpha$)
dans laquelle X est une partie de la liaison ou représente l'une quelconque des séquences d' acides aminés suivantes:

```
1) Val-Ser-Ser-Ser-Arg-Thr-Pro-Ser-Asp-Lys-Pro-Val-
   Ala-His-Val-Val;

2) Val-Arg-Ser-Ser-Ser-Arg-Thr-Pro-Ser-Asp-Lys-Pro-
   Val-Ala-His-Val-Val;

3) Val-Lys-Ser-Cys-Thr-Arg-Thr-Pro-Ser-Arg-Lys-Pro-
   Val-Ala-His-Val-Val;

4) Val-Arg-Ser-Ser-Thr-Arg-Thr-Pro-Ser-Arg-Lys-Pro-
   Val-Ala-His-Val-Val; et

5) Val-Arg-Ser-Cys-Thr-Arg-Thr-Pro-Ser-Arg-Lys-Pro-
   Val-Ala-His-Val-Val.
```

**2.** Procédé de préparation d'ADN codant pour la séquence d'acides aminés suiante:

(la séquence d'acides aminés de TH$\beta_4$)-Asp-Pro-x-(la séquence d'acides aminés correspondant à la séquence de bases construite en liant la guanine avec le début de la séquence de bases du quatrième exon de TNF-$\alpha$)

dans laquelle X est une partie de la liaison ou représente l'une quelconque des séquences d'acides aminés suivantes :

```
1) Val-Ser-Ser-Ser-Arg-Thr-Pro-Ser-Asp-Lys-Pro-Val-
   Ala-His-Val-Val;
2) Val-Arg-Ser-Ser-Ser-Arg-Thr-Pro-Ser-Asp-Lys-Pro-
   Val-Ala-His-Val-Val;
3) Val-Lys-Ser-Cys-Thr-Arg-Thr-Pro-Ser-Arg-Lys-Pro-
   Val-Ala-His-Val-Val;
4) Val-Arg-Ser-Ser-Thr-Arg-Thr-Pro-Ser-Arg-Lys-Pro-
   Val-Ala-His-Val-Val;  et
5) Val-Arg-Ser-Cys-Thr-Arg-Thr-Pro-Ser-Arg-Lys-Pro-
   Val-Ala-His-Val-Val.
```

caractérisé en ce qu'il comprend l'une quelconque des étapes suivantes:

1) l'ADN codant pour (la séquence d'acides aminés de TH$\beta_4$)-Asp-Pro- est lié avec l'ADN codant pour x-(la séquence d'acides aminés correspondant à la séquence de bases construite en liant la guanine avec le début de la séquence de bases du quatrième exon de TNF-$\alpha$);

2) l'ADN codant pour (la séquence d'acides aminés de TH$\beta_4$) est lié à l'ADN codant pour Asp-Pro-x-(la séquence d'acides aminés correspondant à la séquence de bases contruite en liant la guanine avec le début de la séquence de bases du quatrième exon de TNF-$\alpha$): ou

3) l'ADN codant pour (la séquence d'acides aminés de TH$\beta_4$), l'ADN codant pour Asp-Pro et l'ADN codant pour x-(la séquence d'acides aminés correspondant à la séquence de bases construite en liant la guanine avec le début de la séquence de bases du quatrième exon de TNF-$\alpha$) sont liés entre eux simultanément dans leur ordre.

**3.** Plasmide qui incorpore l'ADN codant pour la séquence d'acide aminés suivante:

(la séquence d'acides aminés de TH$\beta_4$)-Asp-Pro-x-(la séquence d'acides aminés correspondant à la séquence de bases construite en liant la guanine avec le début de la séquence de bases du quatrième exon de TNF-$\alpha$)

où X est une partie de la liaison ou représente l'une quelconque des séquences d'acides aminés suivantes:

```
1) Val-Ser-Ser-Ser-Arg-Thr-Pro-Ser-Asp-Lys-Pro-Val-
   Ala-His-Val-Val;
2) Val-Arg-Ser-Ser-Ser-Arg-Thr-Pro-Ser-Asp-Lys-Pro-
   Val-Ala-His-Val-Val;
3) Val-Lys-Ser-Cys-Thr-Arg-Thr-Pro-Ser-Arg-Lys-Pro-
   Val-Ala-His-Val-Val;
4) Val-Arg-Ser-Ser-Thr-Arg-Thr-Pro-Ser-Arg-Lys-Pro-
   Val-Ala-His-Val-Val;  et
5) Val-Arg-Ser-Cys-Thr-Arg-Thr-Pro-Ser-Arg-Lys-Pro-
   Val-Ala-His-Val-Val.
```

**4.** Polypeptide représenté par la séquence d'acides aminés suivantes:

(la séquence d'acides aminés de THβ₄)-Asp-Pro-x-(la séquence d'acides aminés correspondant à la séquence de bases construite en liant la guanine avec le début de la séquence de bases du quatrième exon de TNF-α)

où X est une partie de la liaison ou représente l'une quelconque des séquences d'acides aminés suivantes:

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; et

5) Val-Arg-Ser-Cys-Thr-Arg-Thr-Pro-Ser-Arg-Lys-Pro-Val-Ala-His-Val-Val.

**5.** Procédé de préparation d'un polypeptide représenté par la séquence d'acides aminés suivante:

(la séquence d'acides amines de THβ₄)-Asp-Pro-x-(la séquence d'acides aminés correspondant à la séquence de bases construite en liant la guanine avec le début de la séquence de bases du quatrième exon de TNF-α)

où X est une partie de la liaison ou représente l'une quelconque des séquences d'acides aminés suivantes:

1) Val-Ser-Ser-Ser-Arg-Thr-Pro-Ser-Asp-Lys-Pro-Val-Ala-His-Val-Val;

2) Val-Arg-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; et

5) Val-Arg-Ser-Cys-Thr-Arg-Thr-Pro-Ser-Arg-Lys-Pro-Val-Ala-His-Val-Val.

caractérisé en ce qu'on cultive un microorganisme qui incorpore un plasmide capable de croître dans le microorganisme hôte et en ce qu'on incorpore de l'ADN qui code pour la séquence d'acides aminés ci-dessus.

**6.** Agent anti-tumoral comprenant un polypeptide représenté par la séquence d'acide aminés suivante:

(la séquence d'acides aminés de THβ₄)-Asp-Pro-x-(la séquence d'acides aminés correspondant à la séquence de bases construite en liant la guanine au début de la séquence de bases du quatrième exon de TNF-α)

où X est une partie de la liaison ou représente l'une quelconque des séquences d'acides aminés suivantes:

```
1) Val-Ser-Ser-Ser-Arg-Thr-Pro-Ser-Asp-Lys-Pro-Val-
   Ala-His-Val-Val;
2) Val-Arg-Ser-Ser-Ser-Arg-Thr-Pro-Ser-Asp-Lys-Pro-
   Val-Ala-His-Val-Val;
3) Val-Lys-Ser-Cys-Thr-Arg-Thr-Pro-Ser-Arg-Lys-Pro-
   Val-Ala-His-Val-Val;
4) Val-Arg-Ser-Ser-Thr-Arg-Thr-Pro-Ser-Arg-Lys-Pro-
   Val-Ala-His-Val-Val;  et
5) Val-Arg-Ser-Cys-Thr-Arg-Thr-Pro-Ser-Arg-Lys-Pro-
   Val-Ala-His-Val-Val.
```

**Revendications pour l'Etat contractant suivant : ES**

**1.** Procédé de préparation d' ADN qui code pour la séquence d'acides aminés suivante:

(la séquence d'acides aminés de TH$\beta_4$)-Asp-Pro-x-(la séquence d'acides aminés correspondant à la séquence de bases construite en liant la guanine avec le début de la séquence de bases du quatrième exon de TNF-$\alpha$)

dans laquelle X est une partie de la liaison ou représente l'une quelconque des séquences d'acides aminés suivantes :

```
1) Val-Ser-Ser-Ser-Arg-Thr-Pro-Ser-Asp-Lys-Pro-Val-
   Ala-His-Val-Val;
2) Val-Arg-Ser-Ser-Ser-Arg-Thr-Pro-Ser-Asp-Lys-Pro-
   Val-Ala-His-Val-Val;
3) Val-Lys-Ser-Cys-Thr-Arg-Thr-Pro-Ser-Arg-Lys-Pro-
   Val-Ala-His-Val-Val;
4) Val-Arg-Ser-Ser-Thr-Arg-Thr-Pro-Ser-Arg-Lys-Pro-
   Val-Ala-His-Val-Val;  et
5) Val-Arg-Ser-Cys-Thr-Arg-Thr-Pro-Ser-Arg-Lys-Pro-
   Val-Ala-His-Val-Val.
```

caractérisé en ce qu'il comprend l'une quelconque des étapes suivantes :

1) l'ADN codant pour (la séquence d'acides aminés de TH$\beta_4$)-Asp-Pro- est lié avec l'ADN codant pour x-(la séquence d'acides aminés correspondant à la séquence de bases construite en liant la guanine avec le début de la séquence de bases du quatrième exon de TNF-$\alpha$);

2) l'ADN codant pour (la séquence d'acides aminés de TH$\beta_4$) est lié à l'ADN codant pour Asp-Pro-x-(la séquence d'acides aminés correspondant à la séquence de bases contruite en liant la guanine avec le début de la séquence de bases du quatrième exon de TNF-$\alpha$); ou

3) l'ADN codant pour (la séquence d'acides aminés de TH$\beta_4$), l'ADN codant pour Asp-Pro et l'ADN codant pour x-(la séquence d'acides aminés correspondant à la séquence de bases construite en liant la guanine avec le début de la séquence de bases du quatrième exon de TNF-$\alpha$) sont liés entre eux simultanément dans leur ordre.

**2.** Procédé de préparation d'un plasmide contenant un ADN codant pour la séquence d'acides aminés suivante:

(la séquence d'acides aminés de TH$\beta_4$)-Asp-Pro-x-(la séquence d'acides aminés correspondant à la séquence de bases construite en liant la guanine avec le début de la séquence de bases du quatrième exon de TNF-$\alpha$)

où X est une partie de la liaison ou représente l'une quelconque des séquences d'acides aminés suivantes:

```
1) Val-Ser-Ser-Ser-Arg-Thr-Pro-Ser-Asp-Lys-Pro-Val-
   Ala-His-Val-Val;
2) Val-Arg-Ser-Ser-Ser-Arg-Thr-Pro-Ser-Asp-Lys-Pro-
   Val-Ala-His-Val-Val;
3) Val-Lys-Ser-Cys-Thr-Arg-Thr-Pro-Ser-Arg-Lys-Pro-
   Val-Ala-His-Val-Val;
4) Val-Arg-Ser-Ser-Thr-Arg-Thr-Pro-Ser-Arg-Lys-Pro-
   Val-Ala-His-Val-Val;   et
5) Val-Arg-Ser-Cys-Thr-Arg-Thr-Pro-Ser-Arg-Lys-Pro-
   Val-Ala-His-Val-Val.
```

où ledit ADN est incorporé dans un ADN vecteur avec un promoteur.

3. Procédé de préparation d'un polypeptide représenté par la séquence d'acides aminés suivante:

(la séquence d'acides aminés de TH$\beta_4$)-Asp-Pro-x-(la séquence d'acides aminés correspondant à la séquence de bases construite en liant la guanine avec le début de la séquence de bases du quatrième exon de TNF-$\alpha$)

où X est une partie de la liaison ou représente l'une quelconque des séquences d'acides aminés suivantes:

```
1) Val-Ser-Ser-Ser-Arg-Thr-Pro-Ser-Asp-Lys-Pro-Val-
   Ala-His-Val-Val;
2) Val-Arg-Ser-Ser-Ser-Arg-Thr-Pro-Ser-Asp-Lys-Pro-
   Val-Ala-His-Val-Val;
3) Val-Lys-Ser-Cys-Thr-Arg-Thr-Pro-Ser-Arg-Lys-Pro-
   Val-Ala-His-Val-Val;
4) Val-Arg-Ser-Ser-Thr-Arg-Thr-Pro-Ser-Arg-Lys-Pro-
   Val-Ala-His-Val-Val; et
5) Val-Arg-Ser-Cys-Thr-Arg-Thr-Pro-Ser-Arg-Lys-Pro-
   Val-Ala-His-Val-Val.
```

caractérisé en ce qu'on cultive un microorganisme qui incorpore un plasmide capable de croître dans le microorganisme hôte et en ce qu'on incorpore de l'ADN qui code pour la séquence d'acides aminés ci-dessus.

4. Procédé de préparation d'un agent anti-tumoral dans lequel on mélange un polypeptide représenté par la séquence d'acides aminés suivante: (la séquence d'acides aminés de TH$\beta_4$)-Asp-Pro-x-(la séquence d'acides aminés correspondant à la séquence de bases construite en liant la guanine au début de la séquence de bases du quatrième exon de TNF-$\alpha$)

où X est une partie de la liaison ou représente l'une quelconque des séquences d'acides aminés suivantes:

1) Val-Ser-Ser-Ser-Arg-Thr-Pro-Ser-Asp-Lys-Pro-Val-**Ala**-His-Val-Val;

2) Val-**Arg**-Ser-Ser-Ser-Arg-Thr-Pro-Ser-Asp-Lys-Pro-Val-Ala-His-Val-Val;

3) Val-Lys-Ser-Cys-Thr-Arg-Thr-Pro-Ser-Arg-Lys-Pro-Val-Ala-His-Val-Val;

4) Val-Arg-Ser-Ser-Thr-Arg-Thr-Pro-Ser-Arg-Lys-Pro-Val-Ala-His-Val-Val; et

5) Val-Arg-Ser-Cys-Thr-Arg-Thr-Pro-Ser-Arg-Lys-Pro-Val-Ala-His-Val-Val

avec un support approprié.

29

$\mathcal{Fig. 1}$

Ⓐ
CTCGAGGGCCAGGATGTGGAGAGT
XhoI

GAACCGACATGGCCACACTGACTCTCCTCTCCCTCTCTCCCTCCCTCCAGCAAACCCTCA

Ⓑ
AGCTGAGGGGCAGCTCCAGTGGCTGAACCGCCGGGCCAATGCCCTCCTGGCCAATGGCCT
DdeI

Ⓒ
GGACCTGAGAGATAACCAGCTGGTGGTGCCATCAGAGGGCCTGTACCTCATCTACTCCCA
DdeI

GGTCCTCTTCAAGGGCCAAGGCTGCCCCTCCACCCATGTGCTCCTCACCCACACCATCAG

Ⓓ
CCGCATCGCCGTCTCCTACCAGACCAAGGTCAACCTCCTCTCTGCCATCAAGAGCCCCTG
HinCI

CCAGAGGGAGACCCCAGAGGGGGCTGAGCCAAGCCCTGGTATCAGCCCATCTATCTGGG

AGGGGTCTTCCAGCTGGAGAAGGGTGACCGACTCAGCGCTGAGATCAATCGCCCCGACTA

CCTCGACTTTGCCGAGTCTGGGCAGGTCTACTTTGGGATCATTGCCCTGTGAGGAGGACG

AACATCCAACCTTCCCAAACGCCTCCCCTGCCCCAATCCCCTTATTACCCCCTCCTTCAG

ACACCCTCAACCTCTTCTGGCTCAAAAAGAGAATTGGGGGCTTAGGGTTGGAACCCAAGC

TTAGAACTTTAAGCAACAAGACCACCACTTCGAAACCTGGCATTCAGGATAGTGTGGCCT

GCACAGTGAAGTGCTGGCAACCACTAAGAATTCAAACTGGGCCTCCAGAACTCACTGGG

GCCTACAGCTTTGATCCCTGACATCTGGAATCTGGAGACAAGGGAGCCTTTGGTTCTGGC

Ⓔ
CAGAATGCTGCAG
PstI

EP 0 341 100 B1

Fig. 2

Fig. 3

EP 0 341 100 B1

32

Fig. 4

EP 0 341 100 B1

Fig. 5

Mean tumor diameter (mm)

† : dead

†

Days after tumor inoculation

34

Fig. 6

$\mathcal{F}\dot{\imath}g.\ 7$

Fig. 8